# EUROPÄISCHE PATENTSCHRIFT

(11) **EP 1 287 844 B1**
(45) Veröffentlichungstag und Bekanntmachung des Hinweises auf die Patenterteilung: **02.01.2004**
(21) Anmeldenummer: 02017496.7
(22) Anmeldetag: 06.08.2002
(51) Int. Cl.: A61M 25/00, A61M 27/00

(54) **Drainage und Lyse Kathetersystem**
Drainage and Lyse Catheter system
Systéme de catheter pour drainage et Lyse

(30) Priorität: 16.08.2001 DE 20113545 U
(43) Veröffentlichungstag der Anmeldung: 05.03.2003
(73) Patentinhaber: REHAU AG + Co, 95111 Rehau (DE)
(72) Erfinder: Weisser, Norbert, 77761 Schiltach (DE); Hölper, Bernd, Dr. med., 36093 Künzell (DE)

(56) Entgegenhaltungen:
- WO-A-97/35629
- WO-A-99/10023
- US-A- 5 395 353
- US-A- 6 165 199

## Beschreibung

Die Erfindung betrifft ein Kathetersystem zur Applikation und Drainage von Flüssigkeiten im Hirn, beispielsweise zur Lyse und Drainage bei Stammganglien, Thalamus- oder interzerebralen Blutungen.

In Folge von traumatischen Unfällen, Hypertonus oder Apolexie treten Blutungen im Bereich des Zerebrum, der Ventrikel oder der Schädelbasis auf. Da es bei Blutungen zu vitalen Störungen des gesamten Organismus kommt, die lebensgefährlich sein können, ist eine sofortige Entfernung der Blutung und der damit verbundenen - Zersetzungsprodukte erforderlich.

Stand der Technik und Praxis ist es, dass ein herkömmlicher, einlumiger Silikonkatheter mittels geeigneter Maßnahmen in den Blutungsherd eingeführt wird. Ist die Blutung frisch, wird der Katheter am proximalen Ende mit einem Drainagebeutel verbunden, so dass sich die Blutung spontan entleeren kann. In der Regel sind die Blutungen jedoch einige Stunden alt, so dass zunächst über den Silikonkatheter eine Lysesubstanz verabreicht wird, anschließend der Silikonkatheter für 4 Stunden verschlossen wird, danach erfolgt der Anschluss an einen Drainagebeutel, wobei schließlich das lysierte Blut spontan abläuft.

Dieser Stand der Technik wird beispielsweise beschrieben in "Minimal invasive Neurosurg 2000 MAR; 43(1)" oder "Nervenarzt 1999 Feb.; 70(2); 123 - 130".

Nachteilig bei diesem Stand der Technik ist, dass die Lysesubstanz nicht gezielt in einem definierten Bereich der Blutung appliziert werden kann. Vielmehr fließt bei den herkömmlichen Kathetern die Lysesubstanz dort ins Hirngewebe, wo der geringste Gegendruck herrscht. So werden oftmals schon lysierte Bereiche weiter lysiert, während in die festen Bereiche einer Blutung nur wenig Lysesubstanz injiziert werden kann. Das hat vor allem zur Folge, dass größere Blutungen kaum lysiert und drainiert werden können, andererseits, dass Lysesubstanz unkontrolliert in gesundes Hirngewebe austritt und dort irreversible Schäden verursacht.
Selbst wenn man längere Katheter oder solche mit mehr Austrittsöffnungen einsetzen würde, ließen sich die genannten Nachteile der herkömmlichen Katheter nicht überwinden.

Hier setzt die Aufgabe der Erfindung ein, ein exakt positionierfähiges Kathetersystem bereitzustellen, mit dem sowohl eine gleichmäßige, als auch gezielte Verteilung der Lysesubstanz in der Blutung möglich ist, ohne dass eine unkontrollierte Lyseapplikation im gesunden Hirngewebe zu einer Schädigung führen kann, weiterhin sogar größere Blutungen komplett lysiert und rückstandsfrei drainiert werden können.

Erfindungsgemäß konnte die Aufgabe durch ein Kathetersystem gelöst werden, das besteht aus einem biegesteifen Drainagekatheter und einen darin enthaltenen, torsionsstabilen Lysekatheter mit den im Schutzanspruch 1 beschriebenen Merkmalen, sowie einen Spiralmandrin.

Die mit der Erfindung erzielbaren Vorteile bestehen insbesondere darin, dass gleichermaßen kleine und große Bereiche im Hirn gezielt angesteuert und, falls nötig, auch in einem eng begrenzten Bereich mit Substanzen beaufschlagt werden können, ohne dass gesundes Hirngewebe beeinträchtigt wird.
Diese Ansteuerung des gewünschten Bereiches, beispielsweise der Blutung, erfolgt dadurch, dass die beiden im Lysekatheter vorhandenen Austrittsöffnungen ― durch Drehen und/oder Ziehen des Lysekatheters ― in Überdeckung mit den gewünschten Drainagekatheteröffnungen gebracht werden, wobei dazu die auf beiden Kathetern aufgebrachten Markierungen die Orientierung ermöglichen.

Nachfolgend wird die Erfindung an einem Ausführungsbeispiel, hier im Falle eines intrazerebralen Eingriffs an einer Blutung mit 25 mm Durchmesser, näher erläutert.

Unter sterilen Bedingungen erfolgt der Schnitt in die Kopfhaut und das Spreizen der Schnittränder. Anschließend wird im Schädelknochen das Bohrloch ― im Durchmesser der Dura-Perforation ― frei Hand in Richtung auf das Ziel gesetzt, anschließend der Bolt-Kit eingedreht.

Nach vorhergehender Berechnung der Zielkoordinaten legt man den Drainage- und Lysekatheter in die Führungsschlitten des Stereotaxigerätes ein und schiebt ihn anschließend mittels Trajektor in das distale Ende der intrazerebralen Blutung vor.
Nun erfolgt über CT die Kontrolle zur Erkennung und Ausmessung der Position des Drainagekatheters, wobei man sich am innenliegenden, röntgendichten Lysekatheter orientiert.
Jetzt wird ― unter Beachtung der an beiden Kathetern angebrachten Markierungen ― der innenliegende Lysekatheter so gedreht, dass beide, an seinem distalen Ende eingebrachten Öffnungen mit den beiden äußersten am distalen Ende des Drainagekatheters eingebrachten Öffnungen übereinstimmen. Jetzt erfolgt die Injektion der Lysesubstanz in die zu lysierende Blutung. Anschließend zieht man den Lysekatheter so weit zurück, bis man die 5 mm-Markierung erreicht. Jetzt wird, wiederum unter Beachtung der jeweils angebrachten Markierungen, der Lysekatheter um 90° gedreht und abermals Lysesubstanz injiziert. Die folgenden Schritte sind analog, bis die 25 mm-Blutung komplett mit Lysesubstanz beaufschlagt ist.
Nun entfernt man den Lysekatheter und verschließt den vor Ort verbleibenden Drainagekatheter. Nach 60 Minuten öffnet man den Drainagekatheter und lässt das Lysat sich spontan entleeren.
Nach Beendigung der Therapie werden Katheter und Bolt-Kit entfernt, es erfolgt ein steriler Wundverschluss und das Anlegen des Wundverbandes.

Das erfindungsgemäße Kathetersystem kann neben der Lyse und Drainage von Blutungen auch zur Behandlung weiterer Indikationen im Hirn Verwendung finden, überall dort, wo flüssige oder gelöste Substanzen ins Hirn eingebracht und/oder entfernt werden müssen. Beispielsweise sind hier zu nennen die Drainage von Abszessen und/oder die wiederholte lokale Gabe von Antibiotikas oder Chemotherapeutikas.

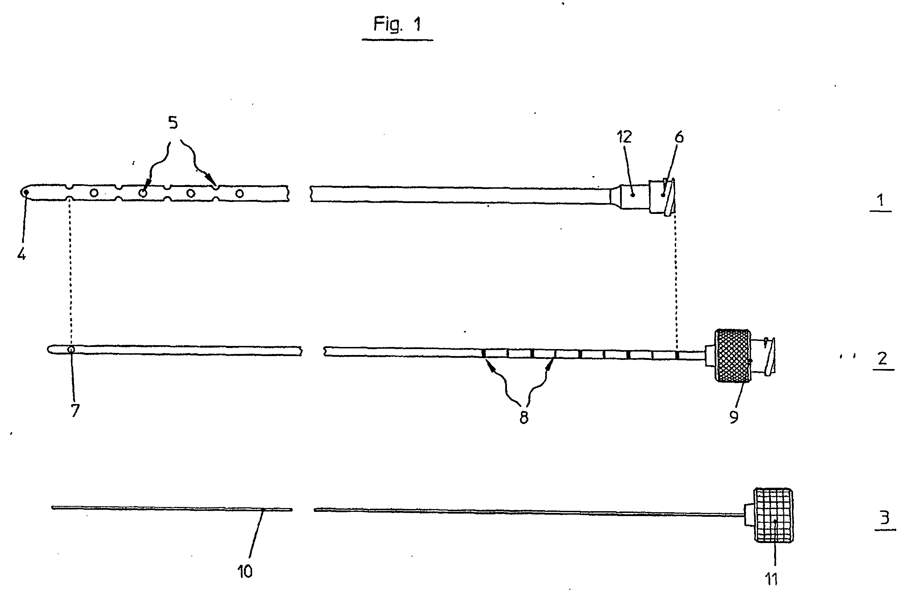

Nachfolgend wird das erfindungsgemäße Kathetersystem im Detail beschrieben. Es umfasst folgende Komponenten, wie in Figur 1 dargestellt:
Der Drainagekatheter 1 besteht aus TPU, er hat eine Länge von 300 mm und einen Außendurchmesser CH9. An seinem distalen Ende ist er mit einer Titankugel 4 versehen, die in den in der Medizin üblichen bildgebenden Verfahren, wie CT oder MRT, sichtbar ist.

Im Anschluss daran sind jeweils im Abstand von 5 mm jeweils zwei einander gegenüberliegende Öffnungen 5 eingebracht, wobei die jeweils folgenden Öffnungen um jeweils 90° versetzt sind. Am proximalen Ende verfügt der Drainagekatheter über einen Luer-Lock-Verschluss weiblich 6 und ein hämostatisches Ventil 12.
Der Lysekatheter 2 besteht aus TPU, er hat eine Länge von 340 mm, einen Außendurchmesser CH5. Er besitzt am distalen Ende zwei einander gegenüberliegende Öffnungen 7. Am proximalen Ende ist ein Luer-Lock-Verschluss männlich/weiblich 9, daran anschließend sind im Abstand von jeweils 5 mm Längenmarkierungen 8, wobei die Markierungen, die 10 mm oder ein Mehrfaches davon sind, durch eine deutlich längere und gleichzeitig auch dickere Markierung gekennzeichnet sind.
Der beschichtete Spiralmandrin 3 hat eine Länge von 350 mm. Er besteht aus dem Spiralmandrin-Körper 10 und - an seinem proximalen Ende - aus einem Griffstück 11, das gleichzeitig als Verschlusskappe männlich des gesamten Kathetersystems dient.

## Patentansprüche

1. Kathetersystem zur Lyse und Drainage bei intraventrikulären, intrazerebralen, subduralen oder zisternalen Blutungen,
**dadurch gekennzeichnet,**
**dass** ein als 1-Lumen-Katheter ausgeführter Lysekatheter mit definierten Öffnungen beweglich in einem als 1-Lumen-Katheter ausgeführten Drainagekatheter mit definierten Öffnungen, eingebracht ist, und dass die im Drainagekatheter und im Lysekatheter definiert eingebrachten Öffnungen durch axiale und/oder tangentiale Bewegung der Katheter gegeneinander, gezielt angesteuert werden.

2. Kathetersystem gemäß Anspruch 1, **dadurch gekennzeichnet, dass** bei der Einführung beider Katheter in die Dura ein zusätzlich eingeführter Spiralmandrin die Armierung bildet.

3. Kathetersystem gemäß Anspruch 1, **dadurch gekennzeichnet, dass** der Lysekatheter am distalen Ende über zwei einander gegenüberliegende Öffnungen, am proximalen Ende über eine Strecke von 40 mm hinweg über Längenmarkierungen, jeweils im Abstand von 5 mm, verfügt.

4. Kathetersystem nach Anspruch 1, **dadurch gekennzeichnet, dass** der Drainagekatheter am distalen Ende über eine Strecke von 40 mm hinweg, jeweils im Abstand von 5 mm, über jeweils zwei einander gegenüberliegende, wechselweise um 90° versetzte Öffnungen verfügt.

5. Kathetersystem nach den Ansprüchen 1 und 3, **dadurch gekennzeichnet, dass** der Lysekatheter aus TPU oder PA-Elastomer besteht und röntgendicht ausgeführt ist.

6. Kathetersystem nach den Ansprüchen 1 und 4, **dadurch gekennzeichnet, dass** der Drainagekatheter zur Lokalisierung in bildgebenden Verfahren am distalen Ende über eine Titankugel verfügt.

## Claims

1. Catheter system for the lysis and drainage of intraventricular, intracerebral, subdural or cisternal haemorrhages,
**characterised by**:
A lysis catheter with defined openings designed as a 1-lumen catheter is inserted movably in a drainage catheter with defined openings designed as a 1-lumen catheter and that the defined openings in the drainage catheter and the lysis catheter can be aligned by axial and/or tangential movement of the catheters relative to each other.

2. Catheter system according to Claim 1, **characterised by**: When the two catheters are inserted into the dura, an additionally inserted spiral mandrin forms the reinforcement.

3. Catheter system according to Claim 1, **characterised by**: The lysis catheter is equipped at the distal end with two opposing openings and at the proximal end with length marks at intervals of 5 mm over a length of 40 mm.

4. Catheter system according to Claim 1, **characterised by**: The drainage catheter is equipped at the distal end with pairs of opposing openings alternating with pairs of openings offset by 90° at intervals of 5 mm over a length of 40 mm.

5. Catheter system according to Claims 1 and 3, **characterised by**: The lysis catheter is made of TPU or PA elastomer and is opaque to X-rays.

6. Catheter system according to Claims 1 and 4, **characterised by**: The drainage catheter is equipped at the distal end with a titanium ball for localisation by imaging methods.

## Revendications

1. Système de cathéters de lyse et de drainage pour les hémorragies intraventriculaires, intracérébrales, sous-durales ou cistemales
**caractérisé en ce**
**qu'**un cathéter de lyse conçu comme un cathéter 1-lumen avec des ouvertures définies est inséré de manière mobile dans un cathéter de drainage conçu comme un cathéter 1-lumen avec des ouvertures définies et que les ouvertures définies pratiquées dans le cathéter de lyse et le cathéter de drainage sont commandées de manière ciblée par un mouvement axial et/ou tangentiel des cathéters l'un par rapport à l'autre.

2. Système de cathéters selon la revendication 1, **caractérisé en ce qu'**un mandrin hélicoïdal également inséré forme l'armature lors de l'introduction des deux cathéters dans la dure-mère.

3. Système de cathéters selon la revendication 1, **caractérisé en ce que** le cathéter de lyse dispose à l'extrémité distale de deux ouvertures opposées et à l'extrémité proximale de repères de longueur avec un écartement de 5 mm sur une section de 40 mm.

4. Système de cathéters selon la revendication 1, **caractérisé en ce que** le cathéter de drainage dispose sur une section de 40 mm de deux ouvertures opposées distantes de 5 mm, décalées de 90°.

5. Système de cathéters selon les revendications 1 et 3, **caractérisé en ce que** le cathéter de lyse est en élastomère TPU ou PA et étanche aux rayons X.

6. Système de cathéters selon les revendications 1 et 4, **caractérisé en ce que** le cathéter de drainage dispose à l'extrémité distale d'une bille en titane servant à la localisation dans un procédé d'imagerie.
